(19) Europäisches Patentamt
European Patent Office
Office européen des brevets

(11) Veröffentlichungsnummer: **0 017 833**
**B1**

(12) # EUROPÄISCHE PATENTSCHRIFT

(45) Veröffentlichungstag der Patentschrift: **20.01.82**

(51) Int. Cl.³: **G 03 C 7/36**

(21) Anmeldenummer: **80101706.2**

(22) Anmeldetag: **31.03.80**

(54) **Farbfotographisches gelbkupplerhaltiges Aufzeichnungsmaterial.**

(30) Priorität: **12.04.79 DE 2915025**

(43) Veröffentlichungstag der Anmeldung:
**29.10.80 Patentblatt 80/22**

(45) Bekanntmachung des Hinweises auf die Patenterteilung:
**20.01.82 Patentblatt 82/3**

(84) Benannte Vertragsstaaten:
**BE DE FR GB**

(56) Entgegenhaltungen:
**FR-A1-2 637 817**
**DE-A1-2 707 488**
**DE-A1-2 723 301**
**US-A-3 730 722**

(73) Patentinhaber: **AGFA-GEVAERT Aktiengesellschaft, Patentabteilung, D-5090 Leverkusen 1 (DE)**

(72) Erfinder: **Renner, Günter, Dr., Roggendorfstrasse 65, D-5000 KÖln 80 (DE)**

EP 0 017 833 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Farbphotografisches gelbkupplerhaltiges Aufzeichnungsmaterial

Die Erfindung betrifft ein farbfotografisches Aufzeichnungsmaterial mit mindestens einer Silberhalogenidemulsionsschicht und einem Gehalt an einem nicht diffundierenden einemulgierten Gelbkuppler, der eine Alkoxyalkylsulfonyl-Gruppe enthält.

Es ist bekannt, farbige fotografische Bilder durch chromogene Entwicklung herzustellen, d.h. dadurch, dass man bildmässig belichtete Silberhalogenidemulsionsschichten in Gegenwart geeigneter Farbkuppler mittels geeigneter farbbildender Entwicklersubstanzen - sogenannter Farbentwickler - entwickelt, wobei das in Übereinstimmung mit dem Silberbild entstehende Oxidationsprodukt der Entwicklersubstanzen mit dem Farbkuppler unter Bildung eines Farbstoffbildes reagiert. Als Farbentwickler werden gewöhnlich aromatische, primäre Aminogruppen enthaltende Verbindungen, insbesondere solche vom p-Phenylendiamintyp, verwendet.

An die Farbkuppler, sowie an die daraus durch chromogene Entwicklung erhaltenen Farbstoffe werden in der Praxis eine Reihe von Forderungen gestellt. So soll die Kupplungsgeschwindigkeit der Farbkuppler mit dem Oxidationsprodukt des Farbentwicklers möglichst gross sein und es soll eine möglichst hohe maximale Farbdichte erzielt werden können. Die Farbkuppler sowie die daraus erhaltenen Farbstoffe müssen hinreichend stabil sein gegenüber Licht, erhöhter Temperatur und Feuchtigkeit. Dies gilt sowohl für frisches Material, als auch für verarbeitetes Material. Beispielsweise darf der in den Bildweissen des verarbeiteten Materials noch vorhandene restliche Kuppler nicht vergilben. Ausserdem sollen die Farbstoffe hinreichend beständig sein gegenüber gasförmigen reduzierenden oder oxidierenden Agentien. Sie müssen ferner diffusionsfest in der Bildschicht verankert sein und sollen sich bei der chromogenen Entwicklung als möglichst feines Korn abscheiden. Schliesslich müssen die aus den Farbkupplern bei der chromogenen Entwicklung entstehenden Farbstoffe eine günstige Absorptionskurve aufweisen mit einem Maximum, das der Farbe des jeweils gewünschten Teilbildes entspricht, und möglichst geringen Nebenabsorptionen.

Ein in der Praxis noch nicht ganz befriedigend gelöstes Problem besteht darin, die Kuppler dauerhaft in fein verteilter Form in die hydrophilen Kolloidschichten von fotografischen Materialien einzubringen, ohne dass sie wieder ausfallen oder in anderer Weise die fotografischen oder mechanischen Eigenschaften der Schichten nachteilig beeinflussen. Hydrophobe Kuppler müssen den Emulsionen in Form von stabilen Dispersionen zugesetzt werden. Bei längerer Lagerung von fotografischen Materialien, die hydrophobe Kuppler enthalten, beobachtet man häufig eine Trübung der Schichten, die auf dem Auskristallisieren des Kupplers beruht. Das Auskristallisieren wirkt sich insbesondere nachteilig aus auf die erzielbare maximale Farbdichte. Gelegentlich gelingt es, die Kristallisationsneigung der Farbkuppler zu verringern durch Einführung bestimmter organischer Reste in das Kupplermolekül, z.B. durch Einführung geeignet substituierter Phenoxygruppen.

In der DE-A-2456076 sind Gelbkuppler beschrieben, die eine Alkylsulfonylamid-Gruppe enthalten und die eine gute Entwicklungsempfindlichkeit aufweisen und bei der Farbentwicklung Farbstoffe mit hoher maximaler Farbdichte und guten Absorptionseigenschaften liefern. Diese Kuppler erfordern jedoch häufig zur Erzielung der genannten hohen maximalen Farbdichte und einer ausreichend hohen Entwicklungsgeschwindigkeit die Anwendung betimmter Entwicklerzusätze wie Benzylalkohol oder Diethylenglykol bei der Farbentwicklung. Ausserdem ist die Herstellung der für ihre Synthese benötigten Alkansulfonsäuren relativ aufwendig.

Der Erfindung liegt die Aufgabe zugrunde, ein farbfotografisches Aufzeichnungsmaterial mit einemulgierten Gelbkupplern anzugeben, die einfach herzustellen sind, leicht emulgiert werden können, eine hohe Reaktivität und gute Empfindlichkeit aufweisen und bei der chromogenen Entwicklung auch ohne Verwendung besonderer Entwicklungszusätze Farbstoffe mit den gewünschten spektralen und sensitometrischen Eigenschaften liefern.

Gegenstand der Erfindung ist ein farbfotografisches Aufzeichnungsmaterial mit mindestens einer lichtempfindlichen Silberhalogenidemulsionsschicht, die einen einemulgierten, nicht diffundierenden Gelbkuppler mit einer Alkylsulfonamidgruppe enthält, dadurch gekennzeichnet, dass der Gelbkuppler der folgenden Formel entspricht

$$Y-NH-SO_2-C_mH_{2m}-O-\left[C_nH_{2n}-O\right]_{p-1}-R \qquad (I)$$

worin bedeuten

Y den Rest eines Acylacetanilid-Gelbkupplers, der den Rest der Formel

$$-NH-SO_2-C_mH_{2m}-O-\left[C_nH_{2n}-O\right]_{p-1}-R$$

in nicht kuppelnder Stelle trägt.

R Wasserstoff oder eine geradkettige oder verzweigte, gesättigte oder olefinisch ungesättigte aliphatische Kohlenwasserstoffgruppe mit 1 bis 20 C-Atomen

m eine ganze Zahl von 1 bis 8, vorzugsweise 2, 3

oder 4
n 2 oder 3
p für eine ganze Zahl von 1 bis 8, vorzugsweise 1, 2 oder 3.

Bei den durch Y dargestellten Gelbkupplern handelt es sich um übliche 4-Äquivalentkuppler wie auch in besonders bevorzugter Weise um 2-Äquivalentkuppler, die in der Kupplungsstelle einen abspaltbaren Substituenten enthalten.

Y lässt sich demnach durch folgende Formel darstellen

$$R^1-CO-\underset{\underset{X}{|}}{CH}-CO-NH-R^2 \qquad (II)$$

worin bedeuten

$R^1$ Alkyl mit bis zu 22 C-Atomen, insbesondere einen verzweigten Alkylrest wie tert.-Butyl, oder Aryl, insbesondere einen Phenylrest, der in für Gelbkuppler üblicher Weise weiter substituiert sein kann, z.B. durch Alkoxy,

$R^2$ Aryl, insbesondere einen Phenylrest, der in für Gelbkuppler üblicher Weise weiter substituiert sein kann, z.B. durch Halogen, Alkoxy,

X Wasserstoff, Halogen, z.B. Chlor oder eine sonstige bei der Kupplung abspaltbare vorzugsweise über Sauerstoff oder Stickstoff angeknüpfte Gruppe.

Erfindungsgemäss ist die Gruppe

$$-NH-SO_2-C_mH_{2m}-O-\left[C_nH_{2n}-O\right]_{p-1}-R$$

entweder über $R^1$ oder über $R^2$ an das Gelbkupplermolekül der obigen Formel (II) angeknüpft. Diese Gruppe kann identisch sein mit der Gruppe, die die Diffusionsfestigkeit des Gelbkupplers bewirkt; sie kann aber auch zusätzlich zu einer solchen Gruppe vorhanden sein.

Als diffusionsfestmachende Reste sind solche Reste anzusehen, die es ermöglichen, die erfindungsgemässen Gelbkuppler in den üblicherweise bei fotografischen Materialien verwendeten hydrophilen Kolloiden diffusionsfest einzulagern. Hierzu sind vorzugsweise organische Reste geeignet, die im allgemeinen gradkettige oder verzweigte aliphatische Gruppen und gegebenfalls auch carbocyclische oder heterocyclische aromatische Gruppen mit im allgemeinen 8–20 C-Atomen enthalten. Mit dem übrigen Molekülteil sind diese Reste entweder direkt oder indirekt, z.B. über eine der folgenden Gruppen verbunden: -NHCO-, $-NHSO_2-$, -NR-, wobei R Wasserstoff oder Alkyl bedeutet, -O- oder -S-.

Da die Diffusionseigenschaften von der Molekülgrösse der verwendeten Gesamtverbindung abhängen, genügt es in bestimmten Fällen, z.B. wenn das verwendete Gesamtmolekül gross genug ist, als «diffusionsfestmachende Reste» auch kürzerkettige Reste zu verwenden.

Sowohl bei dem Rest $-C_mH_{2m}-$ als auch bei dem Rest $-C_nH_{2n}-$ handelt es sich um unverzweigte oder verzweigte Alkylenreste, wobei im letzteren Fall im wesentlichen Methylseitengruppen vorhanden sind.

Beispiele für erfindungsgemässe Gelbkuppler sind im folgenden angegeben:

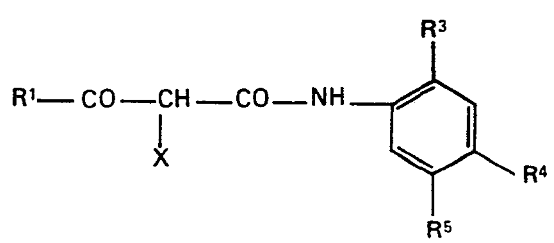

| Kuppl.-No. | $R^1$ | X | $R^3$ | $R^4$ | $R^5$ |
|---|---|---|---|---|---|
| 1 | $(CH_3)_3C-$ | Theoph. | -Cl | H | $NH-SO_2(CH_2)_4-O-C_{12}H_{25}$ |
| 2 | $(CH_3)_3C-$ | Theoph. | -Cl | $-NH-SO_2(CH_3)_4-O-C_8H_{17}$ | H |
| 3 | $(CH_3)_3C-$ | 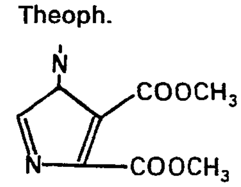 | -Cl | H | $-NH-SO_2-(CH_2)_2-O-C_{14}H_{29}$ |
| 4 | $(CH_3)_3C-$ | 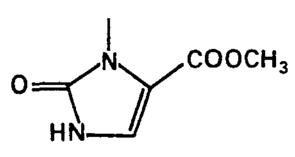 | Cl | $OCH_3$ | $-NH-SO_2-(CH_2)_4-O-C_8H_{17}$ |

Tabelle (Fortsetzung)

| Kuppl.-No. | $R^1$ | X | $R^3$ | $R^4$ | $R^5$ |
|---|---|---|---|---|---|
| 5 | $(CH_3)_3C$- | 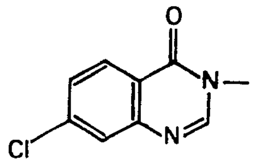 | -Cl | H | $-NH-SO_2-(CH_2)_4-O-CH_2CH_2-O-C_4H_9$ |
| 6 | $(CH_3)_3C$- | 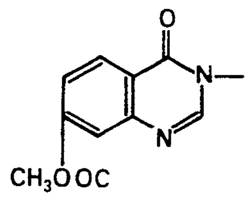 | $-OCH_3$ | H | $-NH-SO_2(CH_2)_4-O-C_{10}H_{21}$ |
| 7 | $(CH_3)_3C$- | 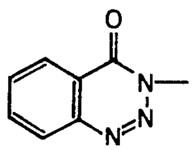 | -Cl | H | $-NH-SO_2-(CH_2)_4-[OCH_2-CH_2]_2-OH$ |
| 8 | $(CH_3)_3C$- | 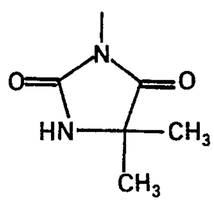 | -Cl | H | $-NH-SO_2(CH_2)_4-O-C_6H_{13}$ |
| 9 | $(CH_3)_3C$- | 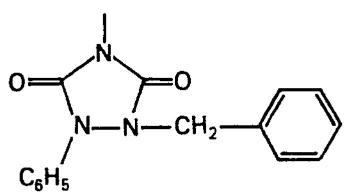 | -Cl | H | $-NH-SO_2(CH_2)_4-O-(CH_2)_8-CH{=}CH-C_8H_{17}$ |
| 10 | $(CH_3)_3C$- | 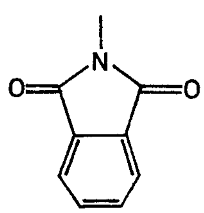 | $-OCH_3$ | $-NH-SO_2(CH_2)_4-O-C_{10}H_{21}$ | H |
| 11 | $(CH_3)_3C$- | 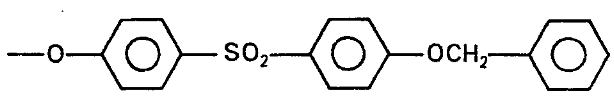 | -Cl | H | $-NH-SO_2-(CH_2)_4-O-(CH_2)_2-O-C_{10}H_{21}$ |
| 12 | $(CH_3)_3C$- | 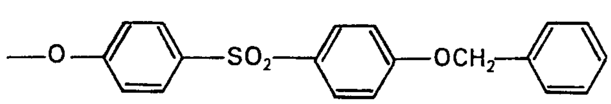 | -Cl | H | $-NH-SO_2-(CH_2)_4-O-C_8H_{17}$ |

Tabelle (Fortsetzung)

| Kuppl.-No. | $R^1$ | X | $R^3$ | $R^4$ | $R^5$ |
|---|---|---|---|---|---|
| 13 | $(CH_3)_3C$- | 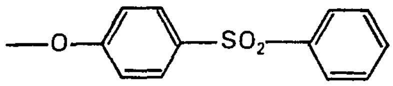 | $-OCH_3$ | H | $-NH-SO_2-CH_2)_4-O-CH_2-CH-C_2H_5$ |
| 14 | $(CH_3)_3C$- | 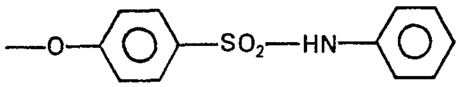 | -Cl | H | $-NH-SO_2(CH_2)_4-O-C_{10}H_{21}$ |
| 15 | $(CH_3)_3C$- | H | -Cl | H | $-NH-SO_2(CH_3)_4-O-C_{12}H_{25}$ |
| 16 | $(CH_3)_3C$- | 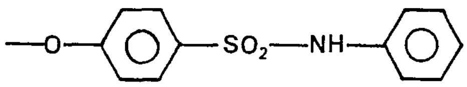 | Cl | $-NH-SO_2(CH_2)_4-O-C_8H_{17}$ | H |
| 17 | $(CH_3)_3C$- | 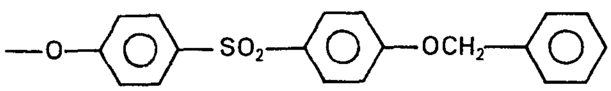 | -Cl | H | $-NH-SO_2-(CH_2)_4-O-C_{12}H_{25}$ |
| 18 | 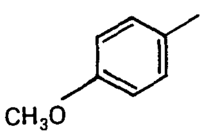 | 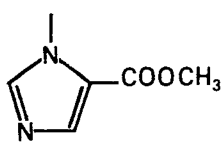 | $-OCH_3$ | H | $-NH-SO_2(CH_2)_4-O-C_{10}H_{21}$ |
| 19 | 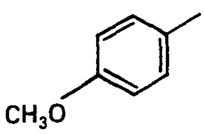 | 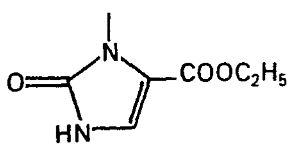 | $-OCH_3$ | H | $-NH-SO_2(CH_2)_4-O-C_{10}H_{21}$ |
| 20 | 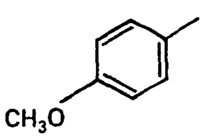 | 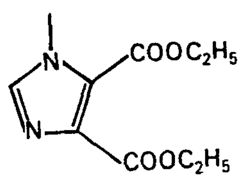 | $-OCH_3$ | $-OCH_3$ | $-NH-SO_2(CH_2)_4-O-C_{10-14}H_{21-29}$ (Isomerengemisch) |
| 21 | 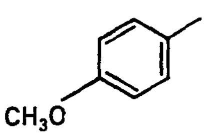 | 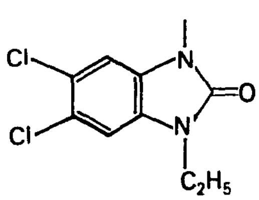 | Cl | H | $-NH-SO_2(CH_2)_4-O-C_8H_{17}$ |
| 22 | 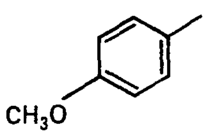 | H | -Cl | H | $-NH-SO_2(CH_2)_4-O-C_8H_{17}$ |
| 23 | 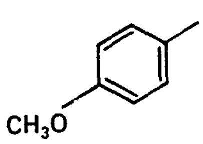 | 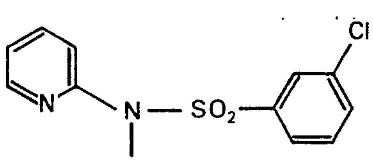 | $-OCH_3$ | $-OCH_3$ | 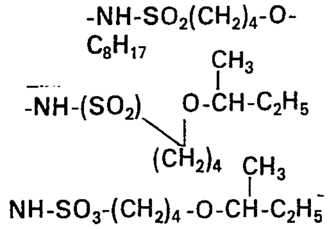 |

Tabelle (Fortsetzung)

| Kuppl.-No. | $R^1$ | X | $R^3$ | $R^4$ | $R^5$ |
|---|---|---|---|---|---|
| 24 | 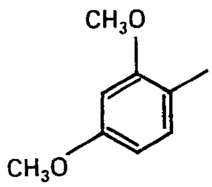 | 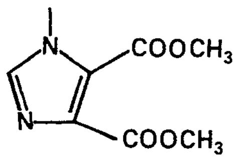 | $-OCH_3$ | $-NH-SO_2-(CH_2)_4-O-(CH_2)_2-O-C_4-H_9$ | $-OCH_3$ |
| 25 | 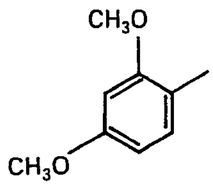 | Theoph. | $-OC_{12}H_{25}$ | H | $-NH-SO_2(CH_2)_4-OCH_3$ |
| 26 | 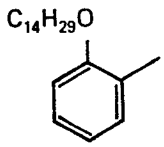 | Theoph. | $-OCH_3$ | H | $-NH-SO_2(CH_2)_4-OCH_3$ |
| 27 | 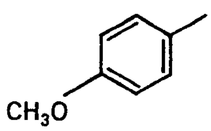 | 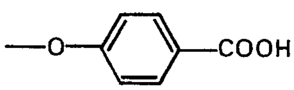 | $-OC_4H_9$ | H | $-NH-SO_2(CH_2)_4-OC_4H_9$ |
| 28 | 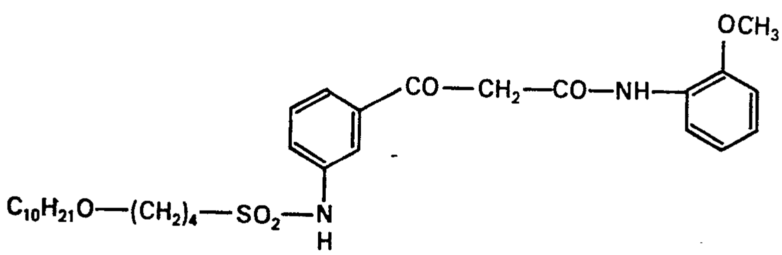 | | | | |
| 29 | 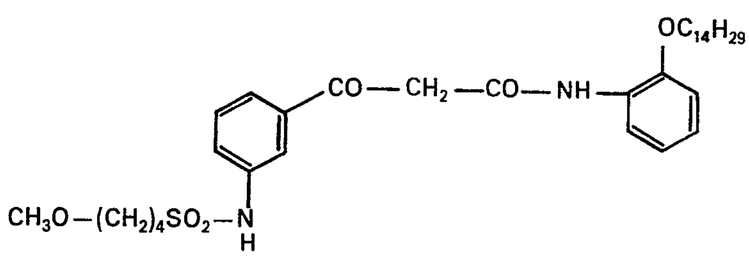 | | | | |
| 30 | 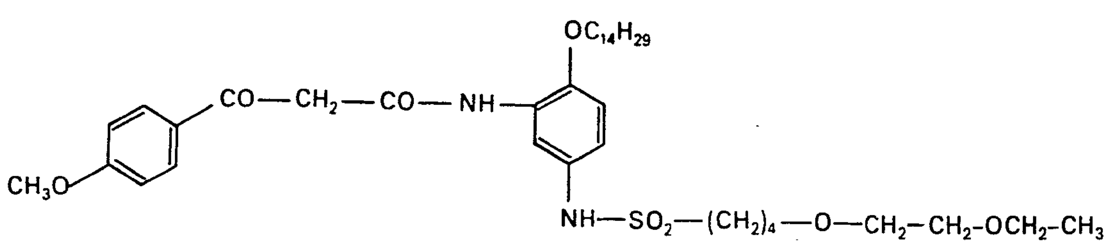 | | | | |

31

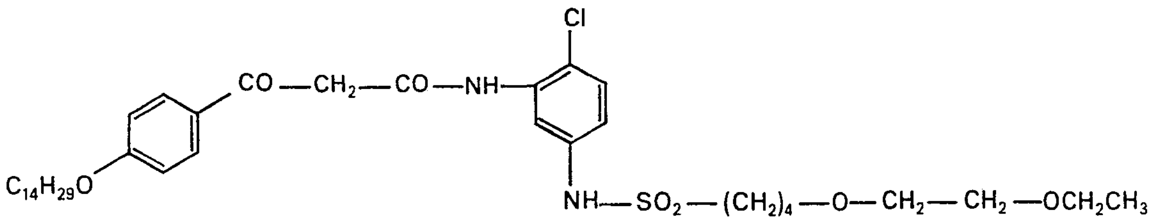

32

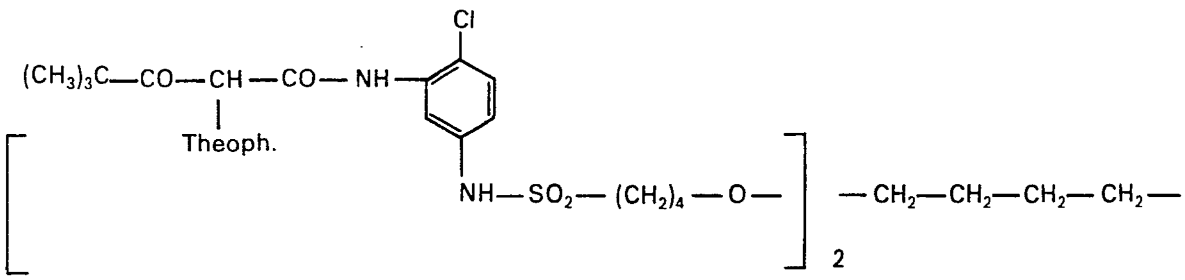

33

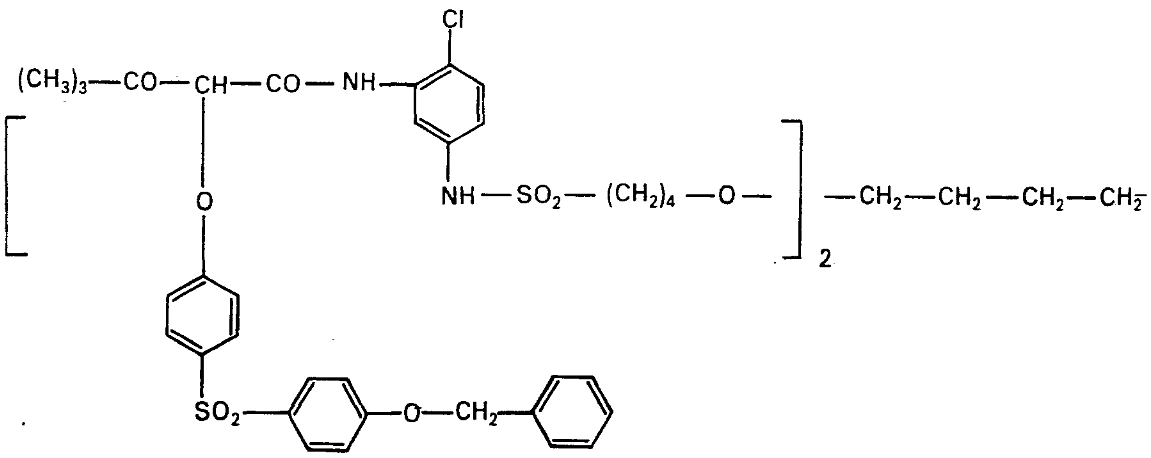

34

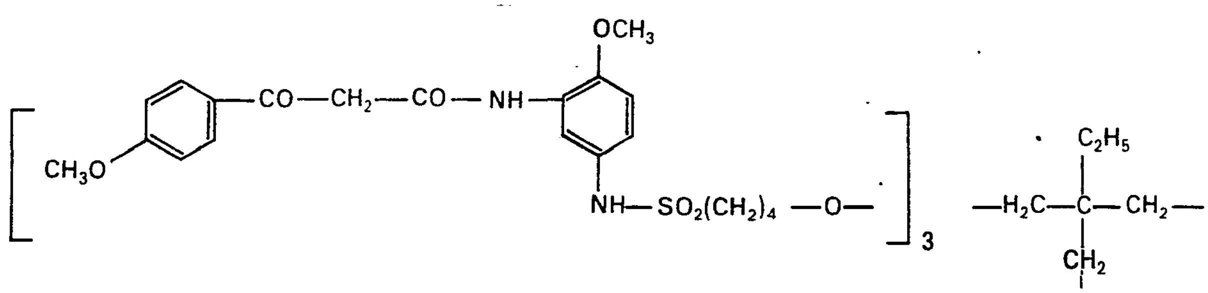

Theoph. = Theophyllin

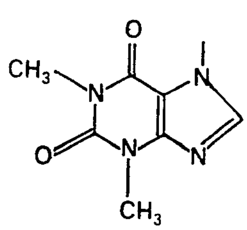

Die erfindungsgemässen Gelbkuppler mit Alkoxyalkylsulfonyl-Rest weisen offenbar aufgrund des Vorhandenseins von mindestens einer Ethergruppierung - mehrere derartige Gruppierungen kumulieren den Effekt - ein zusätzliches hydrophiles Zentrum auf, das die Entwickelbarkeit der Farbkuppler fördert und damit auch ohne die weiter oben genannten Entwicklerzusätze, die als Vermittler zwischen hydrophiler und hydrophober Phase anzusehen sind, die Entwicklung der Farbstoffe aus Farbkuppler mit der geforderten Empfindlichkeit und maximalen Farbdichte ermöglicht. Diese Wirkung der zusätzlichen hydrophilen Zentren (Etherbindungen) hat auch einen positiven Einfluss auf das in DE-A-2 456 076 beschriebene «konkurrierende Reaktionsverhältnis» von Farbkupplern.

Der Ausdruck «konkurrierendes Reaktionsverhältnis» bezieht sich auf das Verhältnis der relativen Reaktionsfähigkeiten eines Gelbkupplers, einerseits in Gegenwart und andrerseits in Abwesenheit eines konkurrierenden Kupplers, wie Zitrazinsäure, H-Säure, Weisskuppler gemäss der folgenden Gliederung

$$K = \frac{D_0}{D_1}$$

$D_0$ = maximale Farbdichte einer fotografischen Schicht, die in einem Farbentwickler ohne konkurrierendem Kuppler entwickelt wurde,

$D_1$ = maximale Farbdichte der gleichen Schicht, die in einem Farbentwickler mit konkurrierendem Kuppler entwickelt wurde.

Der oben beschriebene positive Einfluss der erfindungsgemässen Farbkuppler in fotographischen Schichten äussert sich in einem kleineren konkurrierenden Reaktionsverhältnis. D.h. die erfindungsgemässen neuen Farbkuppler besitzen gegenüber den bekannten alkylsulfonylgruppenhaltigen Farbkupplern den Vorteil, dass sie bei der Farbkupplung durch die Anwesenheit von Konkurrenzkupplern weniger beeinflusst werden. Dies zeigt sich dann in einem kleineren konkurrierenden Reaktionsverhältnis bzw. in einer höheren Farbausbeute bei entsprechender Entwicklung. Die Vorteile einer höheren Farbausbeute, wie beispielsweise geringerer Kuppler- und Silberauftrag, speziell in der Gelbschicht, sind bekannt und bedürfen hier keiner näheren Erläuterung.

Die erfindungsgemässen Gelbkuppler zeichnen sich weiterhin vor allem durch eine ausgezeichnete Löslichkeit und geringe Kristallisationstendenz in organischen Lösungsmitteln, insbesondere in mit Wasser nicht mischbaren Lösungsmitteln mit hohem Siedepunkt, wie z.B. Trikresylphosphat-Isomerengemisch oder Dibutylphthalat aus.

Ausserdem besitzen sie eine hervorragende Diffusionsfestigkeit in fotografischen Schichten, sowohl beim Giessvorgang als auch während der fotografischen Verarbeitung.

Ein besonderer Vorteil der Alkoxyalkylsulfonyl-Ballastgruppen liegt auch darin, dass sie im Vergleich zu langkettigen oder verzweigten, hydrophoben Alkylresten wesentlich verbesserte hydrophile Eigenschaften ergeben. Dies drückt sich u.a. durch hohe Kupplungsaktivität aus. Betreffend Vorteile hydrophiler Gruppen in öllöslichen Kupplern siehe auch die DE-A-1 958 303.

Die Kombination von ausgezeichneter Löslichkeit in organischen Lösungsmitteln und hervorragender Diffusionsfestigkeit einerseits mit guter Wasserverträglichkeit andererseits ist eine besonders überraschende, unvorhersehbare Eigenschaft der erfindungsgemässen Kuppler. Ein weiterer Vorteil der erfindungsgemässen Kuppler liegt in ihrer leichten Herstellbarkeit und in der guten Zugänglichkeit der benötigten Ausgangsprodukte. Die neuen Kuppler ergeben bei der Farbenentwicklung Farbbilder von vorzüglicher Lichtechtheit und günstiger spektraler Absorption, die für die fotografischen Anwendungen geeignet ist.

Das farbfotografische Material, das unter Verwendung eines derartigen neuen Kupplers hergestellt wird, ist daher durch gute fotographische Eigenschaften, gute Farbreproduktion, ausreichende Stabilität vor und nach den Behandlungen und Leichtigkeit seiner Herstellung gekennzeichnet.

Die in der vorliegenden Patentanmeldung beschriebenen Gelbkuppler mit den erfindungsgemässen Alkoxyalkylsulfonyl-Resten werden nach an sich bekannten Methoden hergestellt.

Die Synthese der genannten 2-Äquivalent-Gelbkuppler erfolgt nach den z.B. in DE-A-2 329 587, GB-A-1 420 564, DE-A-2 545 756, DE-A-2 554 321 beschriebenen Methoden aus den entsprechenden 4-Äquivalent-Gelbkupplern. Fluchtgruppen für 2-Äquivalent-Gelbkuppler sind z.B. aus US-A-3 644 498, US-A-3 408 194 und den DE-A-2 414 006, 2 431 480, 2 433 812, 2 344 155, 2 441 779, 2 442 703, 2 448 170, 2 454 741 bekannt.

Die zur Einführung der erfindungsgemässen Alkoxyalkylsulfonyl-Reste geeigneten Verbindungen können auf zwei verschiedenen Wegen einfach erhalten werden:

1. Durch Umsetzung einer Halogenalkansulfonsäure bzw. eines Derivats davon mit dem Salz eines Alkohols der Formel

$$HO-\left[C_nH_{2n}-O\right]_{p-1}-R$$

2. Durch Umsetzung eines Salzes eines Alkohols der unter 1 genannten Formel mit einem Sulton.

Die aus den Umsetzungen 1 bzw. 2 resultierenden Alkoxyalkansulfonsäuren werden anschliessend in bekannter Weise mit aromatischen Aminogruppen eines entsprechenden Gelbkupplers umgesetzt.

Besonders das 2. Verfahren ist aufgrund der einfachen Synthese und der auf diesem Wege sehr kostengünstig darzustellenden Alkoxyalkansulfonsäure und ihrer Derivate als Verfahren

zur Darstellung von Bausteinen für in der Farbenfotografie einzusetzenden Farbkuppler geeignet.

Eine weitere Möglichkeit zur Herstellung der erfindungsgemässen Gelbkuppler besteht in der Umsetzung eines Gelbkupplers mit einer aromatischen Aminogruppe mit einem Halogenalkansulfonsäurehalogenid, z.B. 4-Chlorbutansulfonsäurechlorid zum entsprechenden Sulfonamid, das anschliessend mit dem Salz eines Alkohols zum Ether umgesetzt wird.

Als Alkohol der Formel

$$HO-\left[C_nH_{2n}-O\right]_{p-1}-R$$

kommen beispielsweise folgende Hydroxyverbindungen in Frage:

Monoalkohole:
z.B. $HO\text{-}CH_3$
$HO\text{-}C_2H_5$
$HO\text{-}CH_2\text{-}CH_2\text{-}CH_3$
$HO\text{-}CH\,(CH_3)_2$
$HO\text{-}(CH_2)_3\text{-}CH_3$
$HO\text{-}(CH_2)_3\text{-}CH(CH_3)_2$
$HO\text{-}CH_2\text{-}C(CH_3)_3$
$HO\text{-}C(CH_3)_3$
$HO\text{-}(CH_2)_4\text{-}CH_3$
$HO\text{-}CH(CH_3)\text{-}CH_2\text{-}CH_3$
$HO\text{-}CH_2\text{-}CH(C_2H_5)\text{-}CH_2\text{-}CH_3$
$HO\text{-}(CH_2)_5\text{-}CH_3$
$HO\text{-}(CH_2)_7\text{-}CH_3$
$HO\text{-}(CH_2)_2\text{-}CH(CH_3)\text{-}CH_2\text{-}C(CH_2)_3$

Geradekettige und verzweigte sowie gemischte Fettalkohole mit 10 und mehr C-Atomen.

$HO\text{-}(CH_2)_8\text{-}CH{=}CH\text{-}(CH_2)_7\text{-}CH_3$ u.a. ungesättigte Alkohole, Monoalkylether von Diolen wie Ethylenglykol oder Di-, Tri- oder Tetraalkylenglykol, beispielsweise
$HO\text{-}(CH_2)_2\text{-}O\text{-}C_2H_5$
$HO\text{-}(CH_2)_2\text{-}O\text{-}C_4H_9$
$HO\text{-}[(CH_2)_2O]\text{-}C_4H_9$
$HO\text{-}(CH_2)_2CH(OCH_3\text{-}CH_3$
$HOCH(CH_2\text{-}OC_2H_5)_2$

Diole
$HO\text{-}(CH_2)_n\text{-}OH$ $n = 1\text{-}20$
z.B. $HO\text{-}CH(C_2H_5\text{-}CH_2\text{-}OH$
$HO\text{-}CH(CH_3)\text{-}CH_2\text{-}CH_2\text{-}OH$
$HO\text{-}CH(CH_3)\text{-}CH(CH_3)\text{-}OH$
$HO\text{-}CH_2\text{-}CH{=}CH\text{-}CH_2\text{-}OH$
$HO\text{-}C(CH_3)_2\text{-}CH_2\text{-}CH_2\text{-}C(CH_3)_2\text{-}OH$
Ethylenglykol, Di-, Tri- oder Tetraethylenglykol.

Triole, Tetraole

$CH_2\text{-}OH$
$CH\text{-}OH$
$CH_2\text{-}OH$

$CH_2\text{-}OH$
$HO\text{-}CH_2\text{-}C\text{-}CH_2OH$
$C_2H_5$

$CH_2OH$
$HOCH_2\text{-}C\text{-}CH_2\text{-}OH$
$CH_2\text{-}CH$

Anstelle des 4-Hydroxybutansulfonsäure-Gliedes in den Alkoxyalkansulfonsäureresten der erfindungsgemässen Gelbkuppler, das erhalten wird durch Umsatz mit 1,4-Butansulton, können natürlich auch andere Sultone, z.B. Propansulton, oder 1,3-Butansulton u.a. verwendet werden. Der Einsatz dieser hier nicht durch Beispiele belegten Sultone ist jedoch aus Gründen der Gesundheitsgefährdung problematisch und deshalb bei verantwortungsvollem Arbeiten nur mit hohem technischen Aufwand realisierbar.

Die Herstellung einiger bei der Synthese der erfindungsgemässen Gelbkuppler benötigten Alkoxyalkansulfonsäuren bzw. deren Salze ist im folgenden im Detail beschrieben.

A: $Na\ O\text{-}SO_2\text{-}(CH_2)_4\text{-}OCH_3$
54 g (1,0 Mol) Natriummethylat wird in 300 ml Toluol angeschlämmt. Es wird auf 100°C erwärmt und dann gibt man langsam, wobei das Lösungsmittel aufweicht, 150 g (1,1 Mol) Butansulton zu. Nach 30 Min. Rückflusskochen wird abgekühlt und mit 500 ml Aceton versetzt. Das Natriumsalz der 4-Methoxybutansulfonsäure wird abgesaugt und mit Aceton gewaschen. Man erhält 162 g weisses Produkt (85%).

B: $Na\ OSO_2\,(CH_2)_4\text{-}O\text{-}C_8H_{17}$
128 g (1,0 Mol) Oktanol werden mit 400 ml Toluol gemischt. Dazu fügt man langsam und vorsichtig 24 g (1,0 Mol) Natriumhydrid. Zur Vervollständigung der Salzbildung wird unter Rühren erwärmt, bis zur Beendigung der $H_2$-Entwicklung. Bei 75-80°C fügt man dazu 136 g (1,0 Mol) Butansulton. Bei einer Innentemperatur von 100°C wird nach der Zugabe noch 1 h nachgerührt. Die Suspension wird ohne weitere Aufarbeitung zur weiteren Umsetzung verwendet.

C: $Na\ OSO_2(CH_2)_4\text{-}O\text{-}C_{12}H_{25}$

Man arbeitet analog zu der unter B gegebenen Vorschrift mit der Abänderung, dass anstelle von 24 g NaH 23 g Natrium eingesetzt werden.

D: $(CH_3)_3C\text{-}O\text{-}(CH_2)_4\text{-}SO_3K$

Man arbeitet wie unter A beschrieben mit der Abänderung, dass man Kalium-tert.-butylat an Stelle von Natriummethylat verwendet.

E: $C_{12}H_{25}\text{-}O\text{-}(CH_2)_4\text{-}SO_3Na$

186 g Dodekanol und 54 g Natriummethylat werden auf 140°C erwärmt. Man destilliert Methanol zuletzt im Vakuum ab. Dazu gibt man 150 g Butansulton und erwärmt bis 140°C. Man lässt abkühlen, versetzt mit Essigester und saugt das Natriumsalz ab. Ausbeute: 92%

F: $C_4H_9\text{-}O\text{-}CH_2\text{-}CH_2\text{-}OCH_2\text{-}CH_2\text{-}O\text{-}(CH_2)_4\text{-}SO_3Na$

Nach der unter B angegebenen Methode wird das Natriumsalz des Diethylenglykolmonobutylethers hergestellt. In ebenfalls gleicher Weise wird mit Butansulton umgesetzt. Der verbleibende Rückstand wird mit dem doppelten Volumen Acetonitril versetzt, abgekühlt und abgesaugt. Man erhält 233 g Salz (81%).

G: Na $SO_3$-$(CH_2)_4$-O-$CH_2$-$CH_2$-O-$(CH_2)_4$-$SO_3Na$

Die Darstellung des Dinatrium-Salzes erfolgt analog der in Houben-Weyl VI/2 S. 12 ff beschriebenen Methoden. Man setzt analog B mit doppelt molarer Menge Butansulton um und arbeitet wie unter F beschrieben auf.

H: $CH_3O$-$CH_2$-$CH_2$-$SO_3Na$

166,5 g 2-Chloräthansulfonsäure Natriumsalz werden in 500 ml Dimethylacetamid bei 90°C innerhalb 3 h mit 54 g Natriummethylat unter Rühren umgesetzt. Das Lösungsmittel wird abgedampft und der Rückstand mit 300 ml Acetonitril verrührt. Man erhält in quantitativer Ausbeute ein Gemisch des angegebenen Salzes mit Kochsalz.

I: $CH_3O$-$CH_2$-$CH_2$-$SO_3Na$

Anstelle des unter H verwendeten Dimethylacetamids können 500 ml Acetonitril verwendet werden. Man kocht dann 6 h am Rückfluss und saugt das Gemisch ab.

Die Alkoxyalkansulfonsäurechloride können aus obigen Natriumsalzen auf mehreren Wegen erhalten werden.

I. Man säuert das Natriumsalz an und setzt die freie Alkoxyalkansulfonsäure mit einem Halogenierungsmittel wie z.B. Phosgen, Thionylchlorid, Phosphortrichlorid u.a. um.

II. Man setzt das Natriumsalz der Alkoxyalkansulfonsäure mit einem Phosphorsäurehalogenid z.B. $PCl_5$- oder $PBr_5$ in einem Benzin um und destilliert danach das Lösungsmittel und das entstandene Phosphoroxichlorid ab.

III. Man kann wie unter II. beschrieben verfahren mit der Ausnahme, dass man Thionylchlorid/Dimethylacetamid oder Dimethylformamaid an Stelle des Phosphorsäurehalogenids verwendet.

Darstellung der Gelbkuppler
α-Pivaloyl-α-[4-(4-benzyloxyphenylsulfonyl)-phenoxy]-2-chlor-5-(4-octoxy-butylsulfonamido)-acetanilid (Kuppler Nr. 12)

a. 0,55 Mol des nach B.II erhaltenen Alkoxyalkansulfonsäurechlorids werden mit 0,5 Mol 4-Chlor-3-nitroanilin in einem Lösungsmittelgemisch von 800 ml Aceton und 200 ml Pyridin bei 50°C umgesetzt. Man rührt wässrig aus, nimmt mit Essigester auf und dampft das Lösungsmittel im Vakuum ab. Das Produkt bleibt ölig, Ausbeute nahezu quantitativ.

b. 174 g aus unter a. erhaltenen Produktes werden in 600 ml Methanol gelöst und bei 50°C, 50 bar mit Raney-Nickel als Katalysator hydriert. Man saugt vom Raney-Nickel ab und versetzt den Rückstand mit Salzsäure. Das Anilinhydrochlorid kristallisiert aus und wird abgesaugt. Man erhält 118 g = 66% Ausbeute.

c. 107 g des bei b. erhaltenen Anilins werden in 600 ml Xylol und 100 ml Pyridin einer Aminolyse mit 56 g Pivaloylessigsäureethylester unterworfen.

Das Lösungsmittel wird abdestilliert; der Rückstand kann aus Methanol umkristallisiert werden.

Man erhält 81 g (74% Ausbeute) 4-Äquivalentkuppler vom Schmelzpunkt 92–94°C.

d. 51,6 g des unter c. erhaltenen Kupplers werden mit 13,5 g Sulfurychlorid in 150 ml Methylenchlorid chloriert. Das Lösungsmittel wird entfernt. Der Rückstand wird mit 200 ml Dimethylacetamid versetzt. Dazu gibt man 31 g 4'-Benzyloxy-4-hydroxydiphenylsulfon und 30,5 g Diazabicycloundecen. Man rührt 3 h bei Raumtemperatur, arbeitet sauer auf, nimmt mit Essigester auf, dampft ein und kristallisiert den Rückstand aus Alkohol um. F: 74–76°C, Ausbeute 53 g = 62% (Kuppler 12).

Gelbkuppler Nr. 15 (4-Äquivalentkuppler) und Nr. 1 (2-Äquivalentkuppler)

a. 173 g 2-Chlor-5-nitroanilin werden in 1000 ml Xynol mit 223 g Pivaloylessigester am Rückfluss erhitzt. Xylol wird abdestilliert. Der Rückstand kann aus Äthanol umgelöst werden. Ausbeute 221 g = 74%.

b. 200 g Natriumprodukt nach a. werden in 1800 ml Methanol gelöst und bei 35°C mit Raney-Nickel hydriert. Man säuert das Filtrat mit Salzsäure an und kühlt ab. Es fällt α-Pivaloy-2-chlor-5-aminoacetanilid als Hydrochlorid aus. Man erhält 143 g = 69%.

c. Man stellt 4-Dodecoxybutansulfochlorid nach C.I mit Thionylchlorid her. Das Säurechlorid wird ohne weitere Reinigung bei d. eingesetzt.

d. 152 g des unter b. erhaltenen Aminhydrochlorids werden in 150 ml Pyridin und 300 ml Aceton gelöst. Bei 50°C gibt man dazu unter Rühren 178 g des unter c. erhaltenen Säurechlorids. Man rührt nach und fällt mit Wasser/Salzsäure aus. Das Produkt kann aus Alkohol umgelöst werden. Man erhält Gelbkuppler Nr. 15 (4-Äquivalentkuppler) 132 g = 56% Ausbeute.

e. Das bei d. erhaltene Produkt wird wie bei Kuppler Nr. 12, Stufe d., beschrieben chloriert. 30,5 g α-Chlor-α-pivaloyl-2-chlor-5-(α-dodekanoxy-butylsulfonamido)-acetanilid und 10 g Theophyllin werden in 200 ml N-Methylpyrrolidon gelöst. Dazu tropft man langsam bei 40°C 12 g Tetramethylguanidin. Man arbeitet sauer auf und kristallisiert das Produkt aus Methanol um und erhält Gelbkuppler Nr. 1 mit einer Ausbeute 47 g = 62%.

Gelbkuppler Nr. 18

a. Entsprechend dem bei Kuppler Nr. 15, Stufen a und b beschriebenen Verfahren stellt man aus p-Methoxybenzoyl-essigester und 2-Methoxy-5-nitroanilin das Hydrochlorid von α-(p-Me-

thoxy-benzoyl)-2-methoxy-5-amino-acetanilid her.

b. Das nach a. hergestellte Produkt wird mit dem nach Methode E. II, aus Natriumdecanolat, Butansulton und Phosphorpentachlorid erhaltenen Säurechlorid in Methylethylketon und Triäthylamin umgesetzt. Man erhält in 59%iger Ausbeute den entsprechenden 4-Äquivalentkuppler.

c. Die Chlorierung des unter b. erhaltenen 4-Äquivalentkupplers erfolgt nach der für Gelbkuppler Nr. 12, Stufe d., beschriebenen Methode. 31,3 g α-(4-Methoxybenzoyl)-α-chlor-2-methoxy-5- (α-decoxy-butylsulfonylamido)-acetanilid werden mit 8,5 g Imidazol-4-carbonsäuremethylester in 300 ml Acetonitril gelöst. Man gibt portionsweise bei 50°C 5,5 g Natriummethylat zu. Man arbeitet sauer auf, nimmt in Essigester auf, dampft ein und kristallisiert den Rückstand aus Acetonitril um. Ausbeute: 16 g = 44%.

Gelbkuppler Nr. 19

Der bei Gelbkuppler Nr. 18, Stufe b., erhaltene 4-Äquivalentkuppler wird in Methylenchlorid bei 0°–5°C mit Brom, gelöst in Eisessig, bromiert. 3,5 g des erhaltenen bromierten Kupplers werden mit 10 g Imidazolin-2-on-4-carbonsäureethylester in 200 ml Hexamethylphosphorsäuretriamid gelöst. Bei Raumtemperatur werden dazu 16 g Diazabicycloundecen getropft. Man rührt noch 2 h bei gleicher Temperatur nach und arbeitet danach nach üblicher Methode auf. Das Produkt kann aus Methanol umgelöst werden. Ausbeute: 23,5 g = 63 %.

Gelbkuppler Nr. 3

Nach der Methode B wird aus Myristinalkohol in Xylol das entsprechende Alkoholat hergestellt. Zu 238 g dieses Natriumalkoholats in 1000 ml Xylol gibt man 190 g 2-Bromethansulfonsäure-Natriumsalz und kocht am Rückfluss. Das Salzgemisch lässt sich absaugen und mit Aceton waschen. Mit $PCl_5$ kann daraus nach Methode II das Säurechlorid der Tetradekanoxyethansulfonsäure erhalten werden.

Auf dem bei Gelbkuppler Nr. 12 beschriebenen Weg erhält man mit Imidazoldicarbonsäuremethylester als Fluchtgruppe den Gelbkuppler Nr. 3. Aus Acetonitril umlösbar F: 72–75°C.

Die erfindungsgemässen Gelbkuppler eignen sich in hervorragender Weise für die Verwendung in lichtempfindlichen Silberhalogenidemulsionsschichten farbfotografischer Ein- oder Mehrschichtenmaterialien. Die Kuppler müssen jedoch nicht unbedingt den lichtempfindlichen Silberhalogenidemulsionsschichten einverleibt sein; es ist vielmehr auch möglich, sie in einer lichtunempfindlichen Bindemittelschicht unterzubringen, die einer lichtempfindlichen Silberhalogenidemulsionsschicht benachbart ist.

Die erfindungsgemässen Gelbkuppler können nach einer der bekannten Methoden einer Silberhalogenidemulsion oder aber auch einem anderen Bindemittelgemisch einverleibt werden. Dies geschieht zweckmässig nach einer der folgenden Arbeitsweisen:

(a) Auflösen des Kupplers in einem wenig wasserlöslichen flüchtigen organischen Lösungsmittel (Kp. z.B. oberhalb 200°C) und Dispergieren der Kupplerlösung entweder unmittelbar in einer fotografischen Emulsion oder Dispergieren der Kupplerlösung zunächst in einem wässrigen Medium und anschliessendes Zugeben der Dispersion zu einer fotografischen Emulsion. Din-butylphthalat, Trikresylphosphat, N,N-Diethylcapronsäureamid, Laurinsäuredimethylamid, Dibutylsulfon oder N,N-Dibutylharnstoff sind Beispiele für derartige organische Lösungsmittel. Verwiesen wird hierzu auf US-A-2 304 939, US-A- 2 304 940, US-A-2 322 027.

(b) Dispersion des erfindungsgemässen Gelbkupplers in Lösungsmitteln vom sogenannten Naturharztyp, d.h. beispielsweise in Lösungsmitteln der aus der US-A-2 284 879 bekannten Art.

(c) Zum Dispergieren des Gelbkupplers wird ein verhältnismässig wasserunlösliches in diesem Fall aber niedrigsiedendes Lösungsmittel verwendet, das jedoch während einer der letzten Stufen entfernt werden soll. Ethylacetat, Methylenchlorid, Diethylcarbonat, Chloroform und Cyclohexanon werden als derartige Lösungsmittel verwendet.

(d) Es wird ein mit Wasser mischbares organisches Lösungsmittel verwendet, das während einer der letzten Stufen entfernt werden oder in dem fotografischen Material verbleiben kann. Beispiele für derartige organische Lösungsmittel sind Dimethylformamid, Dimethylsulfoxid, N-Methylpyrrolidon, Methanol und Ethanol.

(e) Erhitzen und Schmelzen des Kupplers ohne Anwendung eines organischen Lösungsmittels und Dispergieren des geschmolzenen Kupplers direkt in einer fotografischen Emulsion oder in einem wässrigen Medium. Diese Arbeitsweise ist für unterhalb 85°C schmelzende Kuppler geeignet. In einigen Fällen kann man die unter (a), (b), (c) und (d) angeführten organischen Lösungsmittel mischen, um eine gute Dispersion des Kupplers zu erhalten.

(f) Schliesslich können die Kuppler auch in Lösungen von polymerisierbaren Monomeren gelöst werden, worauf die Monomeren, z.B. in Gegenwart von Gelatine, polymerisiert werden, wodurch Dispersionen der Farbkuppler in den Polymeren erhalten werden. Verwiesen wird hierzu beispielsweise auf das aus US-A-2 825 382 bekannte Verfahren.

(g) Weiterhin können die Kuppler auch auf geeigneten Latices ausgefällt bzw. niedergeschlagen werden, beispielsweise in der Form, dass die Farbkuppler in einem mit Wasser mischbaren Lösungsmittel gelöst werden, um dann durch Zusatz eines wässrigen Latex wieder in fein verteilter Form an den Polymerteilchen ausgefällt zu werden (siehe dazu DE-A-2 541 274).

Zur Herstellung einer fotografischen Emulsionsschicht können ausserdem ein oder mehrere Kuppler der angegebenen Formel auch zusammen mit einem oder mehreren bekannten Kupplern angewendet werden.

Als lichtempfindliche Emulsionen eignen sich Emulsionen von Silberhalogeniden wie Silberchlorid, Silberbromid oder Gemischen davon, gegebenenfalls mit einem geringen Gehalt an Silberjodid bis zu 10 Mol-%, in einem der üblicherweise hydrophilen Bindemittel wie einem Protein, insbesondere Gelatine, Polyvinylalkohol, Polyvinylpyrroliden, Cellulosederivaten wie Carboxyalkylcellulose, insbesondere Carboxymethylcellulose, oder Derivaten der Alginsäure.

Die farbkupplerhaltigen Silberhalogenidemulsionsschichten können in üblicher Weise weitere Zusätze enthalten wie chemische Sensibilisatoren, spektrale Sensibilisatoren, Stabilisatoren, UV-Absorber, optische Aufheller, Beschichtungshilfsmittel und Antistatika.

Die Emulsionen können in der üblichen Weise gehärtet sein, beispielsweise mit Metallsalzen wie Zirkonylsulfat oder Chromtriacetat oder aber mit organischen Verbindungen wie Formaldehyd, halogensubstituierten Aldehyden, die eine Carboxylgruppe enthalten, wie Mucobromsäure; Diketonen, Dialdehyden, Methansulfonsäureestern, Epoxyden, heterocyclischen Verbindungen, insbesondere Azinen, wie Triazinen, oder Pyrimidinen mit geeigneten Abgangsgruppen, wie Halogenatome, Alkoxygruppen, Alkylsulfonylgruppen oder Gruppen mit einem quaternären Stickstoffatom; Aziridinverbindungen oder Verbindungen, die eine -CO- oder -$SO_2$- Gruppe aktivierte Doppelbindung enthalten, wie Bisacrylamide und dergleichen.

Die neuen Kuppler reagieren sehr gut mit dem Oxidationsprodukt aller gebräuchlichen Entwickler von der Art des p-Phenylendiamins, wie beispielsweise N,N-Diethyl-p-phenylen-diamin, N,N-Diethyl-3-methyl-p-phenylendiamin, 4-Amino-3-methyl-N-ethyl-N-β (-methansulfonamido) ethylanilin, N-Ethyl-N (β-hydroxyethyl) -p-phenylendiamin, N-Ethyl-N-β-hydroxyethyl-3-methyl-p-phenylendiamin, N-Butyl-N-δ-sulfobutyl-p-phenylen-diamin. Weitere brauchbare Farbentwickler sind beispielsweise beschrieben in J.Am. Chem. Soc. 73, 3100 - 3125 [1951].

Neben den genannten Entwicklerverbindungen können die zur Entwicklung der erfindungsgemässen farbfotografischen Aufzeichnungsmaterialien verwendeten Farbentwickler noch die Entwicklung steuernde Verbindungen, wie beispielsweise Citrazinsäure und dergleichen enthalten.

Das erfindungsgemässe Material kann auch einen optischen Aufheller, z.B. ein Stilbenderivat, enthalten.

Von besonderer Bedeutung sind solche farbfotografischen Aufzeichnungsmaterialien, die aus mindestens 3 spektral verschieden sensibilisierten Emulsionsschichten bestehen die übereinander angeordnet sind. Zur Herstellung derartiger Aufzeichnungsmaterialien kann der Schichtträger zunächst beispielsweise mit einer rotempfindlichen Schicht, anschliessend mit einer grünempfindlichen Schicht und schliesslich mit einer blauempfindlichen Schicht beschichtet werden, und zwar mit oder ohne Carey-Lea-Filterschicht zwischen der blauempfindlichen und der grünempfindlichen Schicht. Die drei verschieden farbsensibilisierten Schichten können dabei jedoch auch in anderer Reihenfolge übereinander angeordnet werden, mit der Ausnahme jedoch, das die Carey-Lea-Filterschicht nicht über der blauempfindlichen Schicht angeordnet werden kann. In vorteilhafter Weise werden die lichtempfindlichen Schichten auf der gleichen Seite des Schichtträgers angeordnet. Gegebenenfalls kann das Aufzeichnungmaterial in üblicher bekannter Weise Zwischen- und/oder Deckschichten aufweisen.

Die Erfindung ist jedoch nicht nur auf farbenfotografische Materialien mit mehreren verschiedenen spektral sensibilisierten silberhalogenidemulsionen beschränkt, sondern betrifft auch farbenfotografische Materialien, die Mischkornemulsionen enthalten.

Die Gelbkuppler gemäss der Erfindung können sowohl im Negativ-Positiv-Prozess eingesetzt werden, als auch im Umkehrprozess.

Beispiel 1

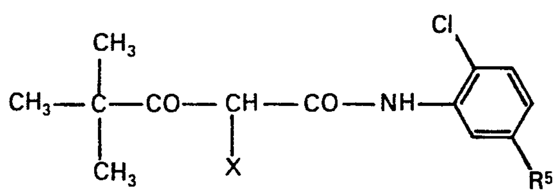

X¹ = 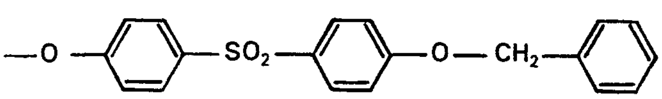

X² = 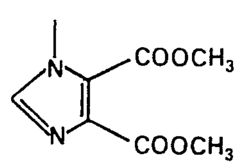

| Kuppler | X | $R^5$ | Fp [°C] |
|---|---|---|---|
| 12 | $X^1$ | $-NH-SO_2-C_4H_8-O-C_8H_{17}$ | 72–75 |
| 17 | $X^1$ | $-NH-SO_2-C_4H_8-O-C_{12}H_{25}$ | 63–65 |
| A | $X^1$ | $-NH-SO_2-C_{16}H_{33}$ | 121–124 |
| 3 | $X^2$ | $-NH-SO_2-C_4H_8-O-C_{14}H_{29}$ | 36–38 |
| B | $X^2$ | $-NH-SO_2-C_{16}H_{33}$ | 92–94 |

Aus der Tabelle ist ersichtlich, dass die erfindungsgemässen Gelbkuppler – verglichen mit entsprechenden Kupplern aus dem Stand der Technik – niedere Schmelzpunkte haben. Das bedeutet, dass die erfindungsgemässen Kuppler leichter emulgierbar sind und stabilere Emulgate liefern.

Vergleichskuppler A:
DE-A-2 456 076 Kuppler Nr. 12
Vergleichskuppler B:
DE-A-2 713 022 Kuppler Nr. 14

Beispiel 2

Jeweils 2 m Mol der in Beispiel 1 erwähnten Kuppler wurden in 3 ml Essigsäureethylester gelöst und nach Zusatz von 1 g Dibutylphthalat bei 60°C unter Verwendung von 0,16 g Natriumdodecylbenzolsulfat in bekannter Weise in 20 ml einer 5%igen wässrigen Gelatinelösung emulgiert. Für die so erhaltenen Emulgatoren wurden nach der in DE-A-2 704 797 beschriebenen Methode die effektiven Reaktionsgeschwindigkeitskonstanten k [$1 . Mol^{-1} . sec^{-1}$] bestimmt.

Folgende Lösungen wurden verwendet:

Entwicklerlösung:

1,12 g Ethylendiamintetraessigsäure-Natriumsalz
11,3 g $Na_3PO_4$
2,5 g $Na_2SO_3$
6,17 g 4-Amino-3-methyl-N-ethyl-N- [β- (methansulfonamido) -ethyl] -anilin-sesquisulfat, auffüllen mit Wasser auf 2000 ml.

Pufferlösung
700 ml 0,1 m $Na_3PO_4$
800 ml 0,1 m $K_2HPO_4$

Für die Bestimmung der k-Werte wurden 6 ml Entwicklerlösung mit 75 ml Pufferlösung gemischt; pH 11,7. Die Ergebnisse sind in der folgenden Tabelle niedergelegt.

| Kuppler | k [$1. Mol^{-1}. sec^{-1}$] |
|---|---|
| 12 | 3900 |
| 17 | 3500 |
| A | 2600 |
| 3 | 5400 |
| B | 3700 |

Hieraus geht hervor, dass die erfindungsgemässen Kuppler reaktiver sind und um bis zu 50% schneller kuppeln als die entsprechenden Vergleichskuppler aus dem Stand der Technik.

Beispiel 3

Die unter Beispiel 2 erhaltenen Emulgate wurden anschliessend mit 85 g einer 7,5%igen Gelatinelösung, die in dispergierter Form 1,93 g Silberbromid enthält, vermischt und mit Wasser bis zum Erreichen der Giessviskosität verdünnt. Das nach Vergiessen auf einen transparenten Cellulosetriacetat-Schichtträger erhaltene Material wird in mehrere Proben aufgeteilt.

a) Hellentwicklung

Es wird für verschiedene Entwickler ($E_1$, $E_2$) die Abhängigkeit der gebildeten Farbdichte von der Entwicklungszeit ermittelt. Die folgende Tabelle gibt die nach 1 Minute erhaltene Farbdichte in Prozenten, bezogen auf die bei Ausentwicklung erhaltene maximale Farbdichte, an.

| Kuppler | Entwickler E 1 | E 2 |
|---|---|---|
| 12 | 32 | 47 |
| 17 | 29 | 44 |
| A | 27 | 36 |
| 3 | 35 | 42 |
| B | 31 | 35 |

Entwickler E 1:

4-Amino-3-methyl-N-ethyl-N-β-hydroxyethyl-anilin $H_2O$-Sulfat, pH 10,6.

Entwickler E 2:

4-Amino-3-methyl-N-ethyl-N [β- (methansulfonamido) – ethyl] -anilin-sesquisulfat, pH 11,7

Die Ergebnisse zeigen, dass die erfindungsgemässen Kuppler, besonders im Entwickler E 2, schneller zu gelben Farbstoffen kuppeln als die bekannten Kuppler.

b) Dunkelentwicklung:

Ein Teil der Proben wurde hinter einem Graustufenkeil belichtet und in dem nachstehend angegebenen Entwickler einmal mit und einmal ohne Benzylalkohol entwickelt.

Entwickler

| | |
|---|---|
| 800 ml | $H_2O$ |
| 2 g | Nitriloessigsäure |
| 1 g | 1-Hydroxyethan-1,1-diphosphonsäure |
| 3 g | Hydroxylamin-sulfat |
| 3 g | $Na_2SO_3$ sicc. |
| 0,6 g | KBr |
| 4 g | 4-Amino-3-methyl-N-ethyl-N-[β-(methansulfonamido)-ethyl]-anilin-sesquisulfat |
| 35 g | $K_2CO_3$ sicc. |
| (15 ml | Benzylalkohol) |

auffüllen auf 100 ml.

Die erhaltenen Farbdichten bei Entwicklung ohne Benzylalkohol ($D_1$) bzw. mit Benzylalkohol ($D_2$) sowie das daraus gebildete Verhältnis

$$\frac{D_1}{D_2}$$

ist in der folgenden Tabelle dargestellt.

| Kuppler | $D_1$ | $D_2$ | $\frac{D_1}{D_2}$ |
|---|---|---|---|
| 12 | 1,25 | 1,68 | 0,74 |
| 17 | 1,08 | 1,54 | 0,72 |
| A | 0,88 | 1,43 | 0,61 |
| 3 | 0,92 | 1,45 | 0,63 |
| B | 0,78 | 1,38 | 0,57 |

Die Tabelle zeigt, dass die erfindungsgemässen Kuppler bereits in einem benzylalkoholfreien Entwickler zu brauchbaren Farbdichten kuppeln. Dies ist vermutlich auf den hydrophilierenden Einfluss der Ethergruppierung im Emulgierteil zurückzuführen, die die Reaktion zwischen hydrophober und hydrophiler Phase, nämlich die Kupplungsreaktion, durch eine Art Phasentransferwirkung begünstigt. Dieser Effekt ist noch stärker ausgeprägt, d.h.

$$\frac{D_1}{D_2}$$

nähert sich dem Wert 1, bei Kupplern mit mehreren Ethergruppierungen im Emulgierteil.

c) Ein weiterer Teil der wie oben hergestellten Materialien wurde zur Bestimmung des «konkurrierenden Reaktionsverhältnisses» (KRV) verwendet. Die Methode ist in DE-A-2456076 beschrieben. Es wurden folgende KRV-Werte gefunden:

| Kuppler | KRV |
|---|---|
| 12 | 1,07 |
| 17 | 1,14 |
| A | 1,22 |
| 3 | 1,24 |
| B | 1,37 |

Dabei wurde ein Farbentwickler folgender Zusammensetzung verwendet:

Farbentwickler:

| | |
|---|---|
| 800 ml | Wasser |
| 1 g | Ethylendiamintetraessigsäure-Na-Salz |
| 6,9 ml | 85% Phosphorsäure |
| 9,65 g | NaBr |
| 0,03 g | KI |
| 31 ml | 45% KOH |
| 4,5 g | $Na_2SO_3$ |
| 11 g | 4-Amino-3-methyl-N-ethyl-N-[β-(methansulfonamido)-ethyl]-anilino-sesquisulfat |
| 1 g | Dithiaoctandiol |
| (1,25 g | Zitrazinsäure) |

auffüllen mit Wasser auf 1000 ml, pH 11,6.

Die Zitrazinsäure ist nur im Vergleichsentwickler enthalten.

Das Ergebnis zeigt zweierlei:

1. KRV-Werte sind keine kupplerspezifischen Konstanten, sondern sehr stark abhängig von bestimmten Bedingungen. Beispielsweise wird für den Kuppler A in DE-A-2456076 ein KRV-Wert von 1,1 angegeben, wobei allerdings ein Farbentwickler einer anderen Zusammensetzung verwendet wurde.

2. Die erfindungsgemässen Gelbkuppler weisen geringere KRV-Werte auf als die Kuppler der DE-A-2456076; sie inhibieren in erhöhtem Masse die Konkurrenzkupplung der Zitrazinsäure.

Beispiel 4

Wie in Beispiel 3 beschrieben wurden weitere Materialien hergestellt mit dem erfindungsgemässen Gelbkuppler Nr. 15 (4-Äquivalentkuppler) und dem Vergleichskuppler C:

Kuppler C: α-Pivaloyl-2-chlor-5-(n-hexadecansulfonamido)-acetanilid

Die Materialien ebenso wie entsprechende Materialien mit den Kupplern 12, 17 und dem Vergleichskuppler A wurden hinter Graustufenkeilen belichtet und in den folgenden Entwicklern (E 3, E 4) negativ entwickelt.

Entwickler E 3:

| | |
|---|---|
| 800 ml | Wasser |
| 2 g | Natriumhexametaphosphat |
| 4 g | $Na_2SO_3$ |
| 3 g | 4-Amino-3-methyl-N, N-diethyl-anilin-Hydrochlorid |
| 20 g | $Na_2CO_3$ |
| 2 g | KBr |

auffüllen mit Wasser auf 1000 ml

pH 10,6.

Entwickler E 4:

| | |
|---|---|
| 800 ml | Wasser 40-50 °C |
| 2 g | Natriumhexametaphosphat |
| 2 g | $Na_2SO_3$ |
| 5 g | 4-Amino-3-methyl-N-ethyl-N-[β-(methansulfonamido)-ethyl]-anilin-sesquisulfat |
| 50 g | $Na_2CO_3$ |
| 1 g | KBr |

auffüllen mit Wasser auf 1000 ml.

Folgende Ergebnisse wurden erhalten:

| Kuppler | E 3 | | E 4 | |
|---|---|---|---|---|
| | Empf. | D max | Empf. | D max |
| 12 | ± 0 | 2,9 | ± 0 | 2,7 |
| 17 | ± 0 | 2,7 | − 0,3 | 2,6 |
| A | − 1,3 | 2,7 | − 1,6 | 2,4 |
| 15 | − 2,0 | 2,1 | − 4,0 | 1,9 |
| C | − 9,3 | 1,7 | − 8,3 | 1,6 |

Die Empfindlichkeit ist in °DIN angegeben, wobei der Kuppler 12 als Standard angesehen wurde.

Der Vergleich zeigt die hohen Empfindlichkeiten, mit denen die erfindungsgemässen Kuppler zu hohen Farbdichten kuppeln; er zeigt aber auch, dass selbst emulgierbare 4-Äquivalentkuppler dieses Typs in fotografischen Materialien mit Vorteil eingesetzt werden können.

**Patentanspruch**

Farbfotografisches Aufzeichnungsmaterial mit mindestens einer lichtempfindlichen Silberhalogenidemulsionsschicht, die einen einemulgierten nicht diffundierenden Gelbkuppler mit einer Alkylsulfonamidgruppe enthält, dadurch gekennzeichnet, dass der Gelbkuppler der folgenden Formel entspricht

$$Y{-}NH{-}SO_2{-}C_mH_{2m}{-}O{-}\left[C_nH_{2n}{-}O\right]_{p-1}{-}R$$

worin bedeuten

Y den Rest eines Acylacetanilid-Gelbkupplers, der den Rest der Formel

$$-NH{-}SO_2{-}C_mH_{2m}{-}O{-}\left[C_nH_{2n}{-}O\right]_{p-1}{-}R$$

in nicht kuppelnder Stelle trägt.

R Wasserstoff oder eine gradkettige oder verzweigte, gesättigte oder olefinisch ungesättigte aliphatische Kohlenwasserstoffgruppe mit 1 bis 20 C-Atomen

m eine ganze Zahl von 1 bis 8,

n 2 oder 3

p eine ganze Zahl von 1 bis 8.

**Claim**

A colour photographic recording material comprising at least one photosensitive silver halide emulsion layer which contains an emulsified non-diffusing yellow coupler containing an alkyl sulphonamide group, characterised in that the yellow coupler corresponds to the following formula

$$Y{-}NH{-}SO_2{-}C_mH_{2m}{-}O{-}\left[C_nH_{2n}{-}O\right]_{p-1}{-}R$$

in which

Y represents the residue of an acyl acetanilide yellow coupler which contains the radical of the formula

$$-NH{-}SO_2{-}C_mH_{2m}{-}O{-}\left[C_nH_{2n}{-}O\right]_{p-1}{-}R$$

in a non-coupling position,

R represents hydrogen or a straight or branched chain, saturated or olefinically unsaturated aliphatic hydrocarbon group containing from 1 to 20 carbon atoms,

m is an integer from 1 to 8,

n is the number 2 or 3, and

p is an integer of from 1 to 8.

**Revendication**

Matériau d'enregistrement photographique couleur comportant au moins une couche d'émulsion d'halogénure d'argent sensible à la lumière, qui contient un coupleur pour jaune non diffusible émulsionné ayant un groupe alkylsulfonamide, caractérisé en ce que le coupleur pour jaune correspond à la formule suivante

$$Y{-}NH{-}SO_2{-}C_mH_{2m}{-}O{-}\left[C_nH_{2n}{-}O\right]_{p-1}{-}R$$

dans laquelle

Y représente le reste d'un coupleur pour jaune d'acylacétanilide, qui porte le reste de formule

$$-NH{-}SO_2{-}C_mH_{2m}{-}O{-}\left[C_nH_{2n}{-}O\right]_{p-1}{-}R$$

dans une position autre que la position de copulation,

R représente l'hydrogène ou un groupe hydrocarboné aliphatique saturé ou oléfinique en $C_1$-$C_{20}$,

m est un nombre entier de 1 à 8,

n est égal à 2 ou 3

p est un nombre entier de 1 à 8.